# EUROPEAN PATENT APPLICATION

(11) **EP 3 127 892 A1**
(43) Date of publication of application: **08.02.2017**
(21) Application number: 15772991.4
(22) Date of filing: 26.03.2015
(51) Int. Cl.: C07C 2/76, C07C 15/04, C07C 15/06, C07C 15/08, C07C 15/24, C01B 3/02, C01C 1/02, C01C 1/04, C07B 61/00

(54) **SYSTEM FOR MANUFACTURING AROMATIC COMPOUND AND METHOD FOR MANUFACTURING SAME**

(30) Priority: 31.03.2014 JP 2014072579
(71) Applicant: Mitsubishi Heavy Industries, Ltd., Tokyo 108-8215 (JP)
(72) Inventor: HAGIMOTO, Akiyori, Tokyo 108-8215 (JP); HIRAYAMA, Haruaki, Tokyo 108-8215 (JP); TANAKA, Yukio, Tokyo 108-8215 (JP); YOSHIOKA, Hiroshi, Tokyo 108-8215 (JP)
(74) Representative: Studio Torta S.p.A.
(86) International application number: PCT/JP2015/059330
(87) International publication number: WO 2015/151992

(57) **Abstract**

A system (1, 1A) for manufacturing an aromatic compound according to the present invention includes: a first manufacturing device (2) that synthesizes a target substance from natural gas; a second manufacturing device that synthesizes an aromatic compound by a catalytic reaction from the natural gas and supplies a mixed gas mainly including unreacted methane and by-product hydrogen to the first manufacturing device (2) to manufacture the target substance; and a hydrogen separation device (3, 3A) that separates hydrogen from purge gas generated from the first manufacturing device (2) and supplies the same to the second manufacturing device (4, 4A) to regenerate the catalyst used for the catalytic reaction.

## Description

### Technical Field

The present invention relates to a system for manufacturing an aromatic compound and a method for manufacturing the same, and particularly relates to a system for manufacturing an aromatic compound in an ammonia manufacturing plant and a method for manufacturing this aromatic compound.

### Background Art

Conventionally, purge gas containing unreacted substances such as hydrogen (H₂), methane (CH₄), and the like is generated at plants where ammonia, methanol, and the like is manufactured from natural gas. As a usage method for such purge gas, a method is known in which, in methanol manufacturing plants, a portion of the hydrogen in the purge gas is recovered and reused and the unrecovered hydrogen is reacted with sulfur and removed as hydrogen sulfide (Patent Document 1).

However, as a method for synthesizing an aromatic compound from natural gas, a method exists in which an aromatic compound can be directly synthesized from the lower hydrocarbons contained in natural gas using a catalytic reaction of zeolite or the like. In such a synthesis method, gas containing unreacted lower hydrocarbon and by-product hydrogen is generated; and methods are known as usage methods for such gas such as a method in which the lower hydrocarbon and the hydrogen are separated and the unreacted gas is reused, and a method in which the gas is methanated and reused (Patent Documents 2 and 3).

### Citation List

### Patent Documents

- Patent Document 1:: Japanese Patent No. 4781612B
- Patent Document 2:: Japanese Patent No. 4565277B
- Patent Document 3:: Japanese Patent No. 5082254B

### Summary of the Invention

### Technical Problem

In light of the foregoing, an object of the present invention is to provide a system for manufacturing an aromatic compound and a method for manufacturing this aromatic compound whereby the purity of surplus hydrogen in purge gas generated in a plant for manufacturing ammonia, methanol, or the like is increased and used to regenerate a catalyst used in the synthesis of an aromatic compound; unreacted gas from the synthesis of the aromatic compound is used in a plant for manufacturing ammonia, methanol, or the like; and by juxtaposing the plant for manufacturing ammonia, methanol, or the like with an aromatic compound manufacturing device, the aromatic compound, and ammonia, methanol, or the like are efficiently co-produced.

### Solution to Problem

A system for manufacturing an aromatic compound of the present invention for achieving the object described above includes a first manufacturing device that synthesizes a target substance from natural gas; a second manufacturing device that synthesizes an aromatic compound from the natural gas via a catalytic reaction, and supplies a mixed gas mainly including unreacted methane and by-product hydrogen to the first manufacturing device to manufacture the target substance; and a hydrogen separation device that separates hydrogen from purge gas generated from a synthesis reaction of the first manufacturing device, and supplies the hydrogen to the second manufacturing device to regenerate a catalyst used in the catalytic reaction.

With the system for manufacturing an aromatic compound according to the present invention, the purity of the surplus hydrogen in the purge gas generated from the synthesis of the target substance from natural gas can be increased; the hydrogen can be used in the regeneration of the catalyst used in the synthesis of the aromatic compound; and the unreacted gas from the synthesis of the aromatic compound can be used in the manufacturing on the target substance. As a result, both an aromatic compound and a target substance derived from natural gas can be efficiently co-produced.

In another embodiment of the present invention, the system may further include a third manufacturing device that synthesizes methane via a methanation reaction from carbon dioxide recovered from exhaust gas of the first manufacturing device and the hydrogen supplied from the hydrogen separation device, and supplies the methane to the second manufacturing device as a raw material.

According to this embodiment, methane can be efficiently synthesized from the carbon dioxide discharged into the atmosphere by the manufacturing device that synthesizes the target substance from the natural gas and the surplus hydrogen other than that used in the catalyst regeneration by the hydrogen separation device; and this methane can be used in the synthesis of the aromatic compound. Accordingly, raw material costs can be suppressed, and the aromatic compound and the target substance can be efficiently obtained.

In the system, preferably, the target substance is ammonia; the aromatic compound is one or more types of aromatic compound selected from the group consisting of benzene, toluene, xylene and naphthalene; the first manufacturing device is an ammonia manufacturing plant; and the second manufacturing device is juxtaposed with the ammonia manufacturing plant, and uses an energy source of the plant to manufacture the aromatic compound.

In this case, the various energy sources generated in large scale at the ammonia manufacturing plant can be used in the manufacturing of the aromatic compound. For example, steam generated in the ammonia manufacturing process can be used as a heating medium or as a motive power source of an axle of a rotary machine such as a compressor or the like in the manufacturing of the aromatic compound; cold energy of ammonia can be used in the manufacturing of the aromatic compound; and electric power generated in large scale can be used in the manufacturing of the aromatic compound. Thus, the co-production efficiency of the aromatic compound and the ammonia by an aromatic compound synthesis device and ammonia manufacturing plant is improved.

The second manufacturing device preferably further includes a compressor and a cooler for manufacturing the aromatic compound.

As a result of this configuration, the energy sources generated in large scale at the ammonia manufacturing plant can be used as an energy source to manufacture the aromatic compound. More specifically, the cold energy of a large cooling system at the ammonia manufacturing plant can be used for the cooler in the manufacturing of the aromatic compound. As a result, production costs can be suppressed, and the manufacturing efficiency of the aromatic compound and the ammonia by the aromatic compound synthesis device and the ammonia manufacturing plant is improved.

In the system, preferably, the catalyst is a catalyst formed from a ZSM-5 type zeolite; an internal pressure of the second manufacturing device is not lower than 0.1 MPa and not higher than 3.0 MPa; and an internal temperature of the second manufacturing device is not lower than 700°C and not higher than 900°C.

By using such a catalyst and under such reaction conditions, the conversion rate of the methane at low reaction pressure in the second manufacturing device can be improved, and the aromatic compound can be efficiently obtained. As a result, the co-production efficiency of the aromatic compound and the ammonia by the aromatic compound synthesis device and the ammonia manufacturing plant is improved.

Furthermore, another aspect of the present invention is a method for manufacturing the aromatic compound. The method for manufacturing the aromatic compound of the present invention includes the steps of:
synthesizing a target substance from natural gas using a first manufacturing device;
synthesizing an aromatic compound from the natural gas via a catalytic reaction using a second manufacturing device, and supplying a mixed gas mainly including unreacted methane and by-product hydrogen to the first manufacturing device to manufacture the target substance; and
separating hydrogen from purge gas generated from the first manufacturing device, and supplying the hydrogen to the second manufacturing device to regenerate a catalyst used in the catalytic reaction.

With the method for manufacturing the aromatic compound according to the present invention, high purity surplus hydrogen separated from the purge gas generated from the synthesis of the target substance from the natural gas can be used in the regeneration of the catalyst used in the synthesis of the aromatic compound; and the mixed gas including the unreacted methane and by-product hydrogen from the synthesis of the aromatic compound can be used in the manufacturing of the target substance. As a result, both an aromatic compound and a target substance derived from natural gas can be efficiently co-produced.

### Advantageous Effects of the Invention

In light of the object, according to the present invention, a system for manufacturing an aromatic compound and a method for manufacturing the aromatic compound are provided whereby the purity of surplus hydrogen of purge gas generated as a by-product of a plant is increased and used to regenerate a catalyst used in the synthesis of an aromatic compound; unreacted gas and or by-products from the synthesis of the aromatic compound are used in an ammonia or methanol manufacturing plant; and by juxtaposing the ammonia or methanol manufacturing plant with an aromatic compound manufacturing device, the aromatic compound and product of the ammonia or methanol manufacturing plant are efficiently co-produced.

### Brief Description of Drawing

FIG. 1 is a conceptual drawing explaining a first embodiment of a system for manufacturing an aromatic compound and a method for manufacturing the aromatic compound according to the present invention.
FIG. 2 is a conceptual drawing explaining an aromatic compound manufacturing device of the first embodiment of the system for manufacturing an aromatic compound and the method for manufacturing the aromatic compound according to the present invention.
FIG. 3 is a conceptual drawing explaining a second embodiment of a system for manufacturing an aromatic compound and a method for manufacturing the aromatic compound according to the present invention.
FIG. 4 is a conceptual drawing explaining an aromatic compound manufacturing device of the second embodiment of the system for manufacturing an aromatic compound and the method for manufacturing the aromatic compound according to the present invention.

### Description of Embodiments

Hereinafter, a first embodiment of a system for manufacturing an aromatic compound according to the present invention will be described in detail while referring to the attached drawings.

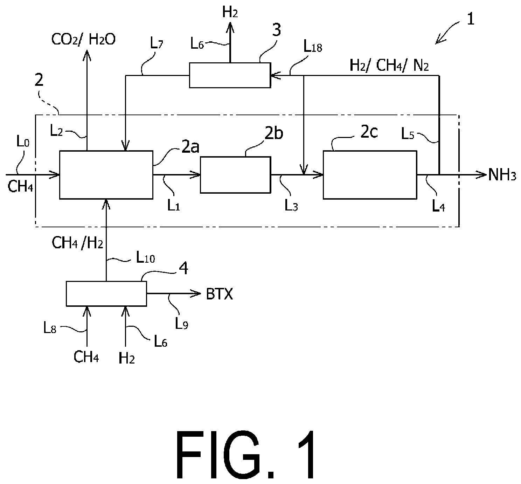

FIG. 1 is a conceptual drawing explaining the first embodiment of the system for manufacturing an aromatic compound according to the present invention. As illustrated in FIG. 1, the system for manufacturing an aromatic compound 1 of the present embodiment includes a manufacturing device 2 (first manufacturing device), a hydrogen separation device 3, and an aromatic compound manufacturing device 4 (second manufacturing device).

The manufacturing device 2 is typically provided in an existing or newly installed ammonia manufacturing plant and is connected to a raw-material supply line L₀, an exhaust gas supply line L₂, a synthesis product supply line L₄, an unreacted gas supply line L₅, a fuel supply line L₇, and a mixed gas supply line L₁₀. The manufacturing device 2 is configured to synthesize ammonia from methane mainly included in natural gas supplied as a raw material from the raw-material supply line L₀, and supply the synthesized ammonia to the synthesis product supply line L₄. In the ammonia synthesis, purge gas is generated as an unreacted material mainly including a large amount of surplus hydrogen, methane, and nitrogen. As such, the manufacturing device 2 is connected to the hydrogen separation device 3 via the unreacted gas supply line L₅ that branches off from the synthesis product supply line L₄, and a purge gas supply line L₁₈ configured to supply the purge gas to the hydrogen separation device 3. The manufacturing device 2 is configured to supply water and carbon dioxide discharged in the ammonia manufacturing process to the exhaust gas supply line L₂. Additionally, the manufacturing device 2 is connected to the aromatic compound manufacturing device 4 via the mixed gas supply line L₁₀ configured to supply mixed gas mainly including methane and hydrogen as fuel from the aromatic compound manufacturing device 4.

The manufacturing device 2 includes a synthesis gas manufacturing device 2a, a first compressor 2b, and a first synthesizer 2c. The manufacturing device 2 can also include a desulfurization device (not illustrated). If such a desulfurization device is provided, the sulfur component included in the raw material natural gas can be adsorption removed in advance, and methane gas having low sulfur concentration can be supplied to both the synthesis gas manufacturing device 2a and the aromatic compound manufacturing device 4 via the raw-material supply line L₀ and a raw-material supply line L₈. Additionally, the manufacturing device 2 includes a large scale cooling system. Due to this cooling system being provided, the cooling system of the manufacturing device 2 can be shared by the manufacturing processes of both the ammonia and the aromatic compound. These configurations contribute to the improvement of the co-production efficiency of the ammonia and the aromatic compound. In addition to the aforementioned, the manufacturing device 2 is configured to use the enormous energy sources of the ammonia manufacturing plant for the aromatic compound manufacturing device 4. Here, "energy sources" are energy sources attributed to large systems of the ammonia manufacturing plant, including the cooling system. Using "the energy sources attributed to large systems" means using energy sources that are used or produced in the ammonia manufacturing process by a large ammonia manufacturing plant. Examples thereof include using a heating medium (e.g. steam) or a cooling medium (e.g. ammonia) as a heat source, using a heating medium (e.g. steam) as a motive power source of a rotary machine, using unreacted materials and/or by-products as fuel, using the large scale electric power, and the like.

The synthesis gas manufacturing device 2a includes a commonly known steam reformer and secondary reformer, and is connected to the raw-material supply line L₀, a process line L₁, the exhaust gas supply line L₂, the fuel supply line L₇, the mixed gas supply line L₁₀, a steam supply line (not illustrated), and an air supply line (not illustrated). The steam reformer is configured to steam-reform the methane supplied from the raw-material supply line L₀ using steam (H₂O) suppled from the steam supply line. The synthesis gas manufacturing device 2a is configured to combust the purge gas introduced via the fuel supply line L₇ and the mixed gas introduced via the mixed gas supply line L₁₀ in a heating furnace (not illustrated), and use the purge gas and mixed gas as fuel for the steam-reforming reaction.

As shown in Formula (1) and Formula (2) below, carbon monoxide (CO), carbon dioxide, and hydrogen are generated from the methane and steam in the steam reformer. The carbon monoxide is converted to carbon dioxide (hereinafter also referred to as "shift reaction"). While the shift reaction is an exothermic reaction, the overall reaction is endothermic so heat must be applied from an external source. The unreacted gas generated in the synthesis process of the aromatic compound in the aromatic compound manufacturing device 4 can be used as the heat from an external source. Accordingly, the co-production efficiency of the ammonia and the aromatic compound can be improved.

[Chemical Formula 1]

CH₄ + H₂O ↔ CO + 3H₂ ...... (1)

CO + H₂O ↔ CO₂ + H₂ ...... (2)

Oxygen supplied from the air supply line reacts with the remaining unreacted methane and a portion of the generated hydrogen in the secondary reformer, and is consumed as shown in Formulas (3) to (5) below. Heat generated by these exothermic reactions is used to produce steam, and this steam is used to produce an energy source and fuel for the steam-reforming reaction. Additionally, the synthesis gas manufacturing device 2a is configured to supply a process gas mainly including nitrogen supplied from the air supply line to synthesize the ammonia and the hydrogen, carbon monoxide, and carbon dioxide generated in the reforming process to the process line L₁; and supply a combustion exhaust gas mainly including carbon dioxide and water to the exhaust gas supply line L₂.

[Chemical Formula 2]

2CH₄ + O₂ → 2CO + 4H₂ ...... (3)

CH₄ + 2O₂ → CO₂ + 2H₂O ...... (4)

2H₂ + O₂ → 2H₂O ...... (5)

Carbon dioxide is selectively removed from the process gas in the synthesis gas manufacturing device 2a by a decarbonization device (not illustrated) disposed on a process line L₃; and, because the trace remaining carbon monoxide becomes a catalytic poison in the ammonia synthesis reaction, this carbon monoxide is methanated and removed via a methanation reaction shown in Formula (6) below.

[Chemical Formula 3]

CO + 3H₂ → CH₄ + H₂O ...... (6)

The first compressor 2b is connected to the process lines L₁ and L₃. The first compressor 2b is configured to compress the process gas supplied from the process line L₁ to a predetermined pressure suited to the synthesis of the ammonia, and supply the compressed process gas to the process line L₃. Note that the process gas compressed by the first compressor 2b may be preheated to a predetermined temperature suited to the synthesis of the ammonia by a heating device (not illustrated) disposed on the process line L₃. Additionally, the unreacted gas supply line L₅ is connected to the process line L₃, and a predetermined amount of the unreacted gas is supplied from the unreacted gas supply line L₅ for the synthesis of the ammonia.

The first synthesizer 2c is connected to the process lines L₃ and the synthesis product supply line L₄. The first synthesizer 2c is configured to synthesize the ammonia from the process gas and/or the unreacted gas supplied from the process line L₃, and supply the ammonia to the synthesis product supply line L₄. A gas mainly including unreacted hydrogen, methane, and nitrogen is generated in the ammonia synthesis reaction in the first synthesizer 2c. As such, the synthesis product supply line L₄ is connected to the unreacted gas supply line L₅ and the purge gas supply line L₁₈ to separate the unreacted gas from the ammonia. Thereby, the synthesis product supply line L₄ is configured to supply the unreacted gas to the unreacted gas supply line L₅, and supply the purge gas to the hydrogen separation device 3 via the purge gas supply line L₁₈. Note that an amount of the purge gas supplied to the purge gas supply line L₁₈ can be adjusted by a supply amount adjustment valve (not illustrated) provided on the purge gas supply line.

The hydrogen separation device 3 is provided juxtaposed with the ammonia manufacturing plant, and is connected to the purge gas supply line L₁₈, a hydrogen supply line L₆, and the fuel supply line L₇. The purge gas supplied at the predetermined amount via the purge gas supply line L₁₈ is supplied to the hydrogen separation device 3 to prevent the accumulation of inert gas. The hydrogen separation device 3 is configured to separate hydrogen from the purge gas supplied from the purge gas supply line L₁₈, and supply the hydrogen to the aromatic compound manufacturing device 4 via the hydrogen supply line L₆. Additionally, the hydrogen separation device 3 is configured to supply a portion of the purge gas from which the hydrogen has been separated to the synthesis gas manufacturing device 2a of the manufacturing device 2 via the fuel supply line L₇ as fuel.

A portion of the hydrogen is separated from the purge gas in the hydrogen separation device 3. Hydrogen partial pressure is higher in the unreacted gas supplied from the unreacted gas supply line L₅ than the mixed gas from the aromatic compound manufacturing device 4. As such, the hydrogen can be efficiently separated from the unreacted gas, which has high hydrogen partial pressure, generated from the manufacturing device 2 of the ammonia manufacturing plant, and used in the catalyst regeneration at the aromatic compound manufacturing device 4.

Examples of hydrogen separation methods at the hydrogen separation device 3 include pressure swing adsorption (PSA) methods, membrane separation methods, and the like.

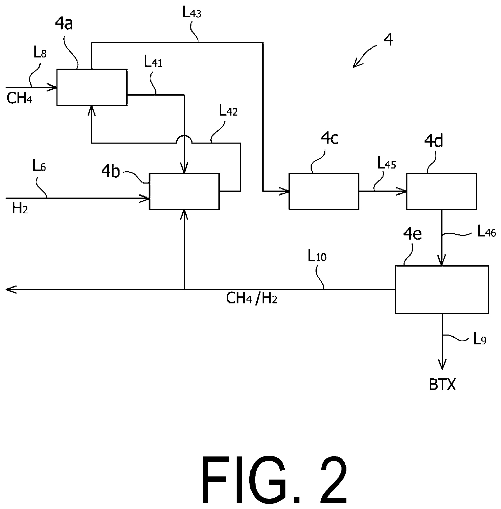

FIG. 2 is a conceptual drawing explaining the aromatic compound manufacturing device 4 of the first embodiment of the system for manufacturing an aromatic compound and the method for manufacturing the aromatic compound according to the present invention. As illustrated in FIG. 2, the aromatic compound manufacturing device (second manufacturing device) 4 is provided juxtaposed with the ammonia manufacturing plant and includes a heater 4a, a second synthesizer 4b, a second compressor 4c, a cooler 4d, and a gas-liquid separator 4e. The aromatic compound manufacturing device 4 is configured to produce an aromatic compound having six or more carbons, particularly benzene (C₆H₆), toluene (C₇H₈), or xylene (C₈H₁₀), from natural gas mainly including a lower hydrocarbon having four or less carbons, namely methane (hereinafter, the one or more aromatic compounds selected from the group consisting of benzene, toluene, and xylene is referred to as "BTX"). The aromatic compound manufacturing device 4 is configured to supply a mixed gas mainly including unreacted methane and by-product hydrogen to the manufacturing device 2, and use this mixed gas as fuel for the ammonia manufacturing process. Additionally, the aromatic compound manufacturing device 4 is configured to remove sediment from the surface of the catalyst used in the aromatic compound, using the hydrogen supplied from the hydrogen separation device 3.

The heater 4a is connected to the raw-material supply line L₈ and process lines L₄₁ to L₄₃. The heater 4a is configured to preheat the natural gas supplied from the raw-material supply line L₈ to a predetermined temperature suited to the synthesis of the aromatic compound, and supply this preheated natural gas to the process line L₄₁. Additionally, from the perspective of manufacturing efficiency, the heater 4a is configured to cause heat exchange to the natural gas from the heat of a process gas with an elevated temperature supplied via the process line L₄₂ from the second synthesizer 4b described hereinafter, use the residual heat of the natural gas, and supply this natural gas to the process line L₄₃. Thereby, the preheating of the fuel supplied from the raw-material supply line L₈ can be efficiently carried out.

The second synthesizer 4b is connected to the process lines L₄₁ and L₄₂, the mixed gas supply line L₁₀, and the hydrogen supply line L₆. The second synthesizer 4b is configured to synthesize an aromatic compound having six or more carbons, particularly BTX, from the methane supplied from the process line L₄₁, and supply the aromatic compound to the process line L₄₂. Additionally, the second synthesizer 4b is configured to combust a portion of the mixed gas including the unreacted methane and the by-product hydrogen supplied via the mixed gas supply line L₁₀ in a heating furnace (not illustrated). Furthermore, the second synthesizer 4b is configured to supply hydrogen gas, which is supplied from the hydrogen supply line L₆, to a catalyst provided therein for catalyst regeneration.

The reaction mechanisms of the reaction in the second synthesizer 4b by which the BTX is synthesized and particularly a methane to benzene (MTB) reaction where benzene is synthesized from methane, includes a reaction mechanism in which carbon (C) is generated as a by-product because the hydrocarbon is subjected to a selective dehydrogenation reaction. The carbon generated in this way mainly deposits and accumulates on the catalyst surface and, as a result, deterioration of the catalyst advances with time, and the catalytic activity thereof declines. As a countermeasure, hydrogen gas for catalyst regeneration is supplied to the second synthesizer 4b from the hydrogen separation device 3. The hydrogen gas is circulated and removes the carbon that has accumulated on the catalyst surface. Thus, declines in the catalytic activity can be prevented by using the supplied hydrogen gas as a catalyst regeneration gas. Note that the catalyst regeneration may be performed simultaneously with the synthesis reaction of the BTX, or may be performed intermittently by stopping the raw material supply from the raw-material supply line L₈ and only supplying the hydrogen.

In the synthesis reaction of the BTX in the second synthesizer 4b and particularly the MTB reaction where benzene is synthesized from methane, an internal pressure of the second synthesizer 4b is preferably not lower than 0.1 MPa and not higher than 3.0 MPa, and an internal temperature is preferably not lower than 600°C and not higher than 1,000°C and more preferably not lower than 700°C and not higher than 900°C. Under these reaction conditions, the manufacturing efficiency of the BTX and the conversion rate of the methane under low reaction pressure can be improved. By obtaining the energy source needed for the BTX manufacturing from the energy source generating facilities of the ammonia manufacturing plant, the facilities will increase in size, and the co-production efficiency of the ammonia and the BTX can be improved.

A catalyst, in which an active metal is supported on a support, is disposed in the second synthesizer 4b. Zeolite, silica, alumina, titania, zirconia, ceria, or a combination of these can be used as the support. Molybdenum (Mo), cobalt (Co), zinc (Zn), gallium (Ga), iron (Fe), copper (Cu), silver (Ag), nickel (Ni), tungsten (W), rhenium (Re), barium (Ba), manganese (Mn), zirconium (Zr), platinum (Pt), ruthenium (Ru), rhodium (Rh), palladium (Pd), or a combination of these can be used as the active metal. The support is preferably a zeolite and more preferably a ZSM-5 type zeolite, and the active metal is preferably molybdenum. With this type of catalyst, the manufacturing efficiency of the BTX and the conversion rate of the methane under low reaction pressure can be improved. As a result, the co-production efficiency of the ammonia and the BTX can be improved.

The second compressor 4c is connected to the process lines L₄₃ and L₄₅. The second compressor 4c is configured to increase the pressure of the process gas supplied from the process line L₄₃ and provide this process gas to the process line L₄₅ in order to separate/recover the liquid phase BTX from the process gas in the gas-liquid separator 4e, preferably such that a recovery rate of the BTX is about 80% or greater. Note that the process gas supplied from the process line L₄₃ has been cooled to a predetermined temperature by a cooler (not illustrated) in advance.

The cooler 4d is configured to further lower the temperature of the process gas supplied from the process line L₄₅ and provide this process gas to the process line L₄₆ in order to separate/recover the liquid phase BTX from the process gas in the gas-liquid separator 4e. An energy source generated at the ammonia manufacturing plant can be used at the second compressor 4c and the cooler 4d. Specifically, from the perspective of co-production efficiency, preferably a portion of the steam produced at the ammonia manufacturing plant is taken as the energy source and diverted to the second compressor 4c. Additionally, preferably a portion of the cold energy obtained by the large cooling system of the ammonia manufacturing plant is taken and diverted to the cooler 4d.

Examples of a cooling medium used in the cooler 4d include organic cooling mediums such as methanol (CH₄O), ethylene glycol (C₂H₆O₂), ammonia (NH₃), and the like; flammable chlorofluorocarbon-based solvents such as HFC-32 (CH₂F₂) and the like; and non-flammable chlorofluorocarbon-based solvents such as HFC-23 (CHF₃), HFC-134a (CH₂FCF₃), HCFC-22 (CHClF₂), HCFC-124 (CHClCF₃), PFC-14 (CF₄), PFC-116 (C₂F₆), PFC-218 (C₃F₈), and the like. From the perspective of manufacturing costs, a product of the juxtaposed plant can be used as the cooling medium. For example, in the present embodiment, because an ammonia manufacturing plant is juxtaposed, ammonia can be used as the cooling medium.

The gas-liquid separator 4e is connected to the process line L₄₆, a BTX supply line L₉, and the mixed gas supply line L₁₀. The gas-liquid separator 4e is configured to separate the process gas supplied from the process line L₄₆ into liquid phase BTX and gas phase mixed gas mainly including unreacted methane and by-product hydrogen, supply the liquid phase BTX to the BTX supply line L₉, and supply the gas phase mixed gas to the mixed gas supply line L₁₀. Note that the mixed gas supply line L₁₀ is configured to branch so that a portion of the mixed gas is supplied to the second synthesizer 4b, and the remainder is supplied to the synthesis gas manufacturing device 2a. Additionally, an amount of the mixed gas supplied to the second synthesizer 4b can be adjusted by a supply amount adjustment valve (not illustrated) provided on the mixed gas supply line L₁₀. Thus, the second manufacturing device 4 is configured to manufacture the BTX and supply the mixed gas generated in the synthesis reaction of the BTX to the first manufacturing device 2 as fuel.

According to the present embodiment, at the ammonia manufacturing plant where ammonia is synthesized from natural gas mainly including methane, the large amount of surplus hydrogen included in the unreacted material, namely the purge gas, of the ammonia synthesis reaction in the manufacturing device 2 can be used for the regeneration of the catalyst used in the catalytic reaction in the aromatic compound manufacturing device 4; and the unreacted gas of the aromatic compound manufacturing device 4 can be used as the fuel for the steam-reforming reaction in the manufacturing device 2. Accordingly, it is possible to synthesize and manufacture both the ammonia and the BTX, and efficiently obtain ammonia and BTX with suppressed manufacturing costs.

Additionally, the energy sources generated at the ammonia manufacturing plant can be used at the second compressor 4c and/or the cooler 4d for the synthesis of the aromatic compound. Thereby, manufacturing costs can be suppressed and the co-production efficiency of the BTX and the ammonia can be improved.

Furthermore, the large cooling system provided in the manufacturing facilities 2 of the ammonia manufacturing plant can be used at the cooler 4d of the aromatic compound synthesis device 4. As a result, manufacturing costs can be suppressed and the co-production efficiency of the BTX and the ammonia can be improved.

Next, by describing the operating configuration of the system for manufacturing an aromatic compound (the first embodiment), a first embodiment of the method for manufacturing the aromatic compound according to the present invention will be described in detail.

The process for manufacturing ammonia from natural gas is described below. As illustrated in FIG. 1, natural gas mainly including methane is supplied from the raw-material supply line L₀ to the manufacturing device 2, and mixed gas mainly including by-product hydrogen and unreacted methane from the aromatic compound manufacturing device 4 juxtaposed with the ammonia manufacturing plant is supplied via the mixed gas supply line L₁₀ to the manufacturing device 2 as fuel. The ammonia synthesized in the manufacturing device 2 is supplied to the synthesis product supply line L₄, and the unreacted gas generated in the ammonia synthesis is supplied to the unreacted gas supply line L₅ that branches off from the synthesis product supply line L₄. A predetermined amount of the unreacted gas is returned from the unreacted gas supply line L₅ to the manufacturing device 2 via the process line L₃ to recycle the raw material, and the remainder is supplied from the unreacted gas supply line L₅ via the purge gas supply line L₁₈ to the hydrogen separation device 3 to prevent the accumulation of inert gas.

The natural gas includes a sulfur component and this sulfur component adversely affects the catalyst. As such, this sulfur component is preferably adsorption removed in advance using a desulfurization device (not illustrated) prior to supplying the natural gas as the raw material. The desulfurization device can manufacture low sulfur concentration natural gas on a large scale as a raw material for the synthesis of the ammonia and the synthesis of the aromatic compound, and can supply this natural gas to both the manufacturing device 2 and the aromatic compound manufacturing device 4 juxtaposed with the ammonia manufacturing plant. At typical ammonia manufacturing plants, natural gas that has not been desulfurized is also used as fuel. As such, the temperature of the exhaust gas decreases and, when the acid component condenses, corrosion problems in the flue occur. In the present invention, only unreacted material and by-product derived from desulfurized natural gas are used as the fuel for ammonia manufacturing. Therefore, the aforementioned problems do not arise and heat recovery from the exhaust gas to lower temperatures is possible. This method contributes to the improvement of the co-production efficiency of the ammonia and the aromatic compound.

Next, a detailed description will be given of a manufacturing process of the ammonia. The natural gas supplied from the raw-material supply line L₀ to the synthesis gas manufacturing device 2a is reformed via a steam-reforming reaction using steam supplied from the steam supply line (not illustrated). Heat required for this reaction is obtained by combusting unreacted gas supplied from the mixed gas supply line L₁₀. At this time, air from the air supply line (not illustrated) is supplied to the first synthesizer 2c to synthesize the ammonia. The oxygen supplied from the air supply line is consumed as shown in Formulas (1) to (3) below. Heat generated by these exothermic reactions is used to produce steam, and this steam is used to produce an energy source and fuel for the steam-reforming reaction. A process gas mainly including nitrogen and the hydrogen generated in the reforming reaction is supplied to the process line L₁, and water and carbon dioxide generated by the combustion of the unreacted gas is supplied to the exhaust gas supply line L₂.

[Chemical Formula 4]

2CH₄ + O₂ → 2CO + 4H₂ ...... (1)

CH₄ + 2O₂ → CO₂ + 2H₂O ...... (2)

2H₂ + O₂ → 2H₂O ...... (3)

As shown in Formula (4) and Formula (5) below, carbon monoxide (CO), carbon dioxide, and hydrogen are generated from the methane and steam in this type of steam-reforming reaction. The carbon monoxide is converted to carbon dioxide in a shift reaction. While the shift reaction is an exothermic reaction, the overall reaction is endothermic so heat must be applied from an external source. The unreacted gas generated in the synthesis process of the aromatic compound in the aromatic compound manufacturing device 4 can be used as the heat from an external source. Accordingly, the co-production efficiency of the ammonia and the aromatic compound can be improved.

[Chemical Formula 5]

CH₄ + H₂O ↔ CO + 3H₂ ...... (4)

CO + H₂O ↔ CO₂ + H₂ ...... (5)

The process gas supplied from the process line L₁ is compressed at the first compressor 2b to a predetermined pressure suited to the synthesis of the ammonia, and the compressed process gas is supplied to the process line L₃. Carbon dioxide is selectively removed from the process gas supplied to the process line L₃; and, because the trace remaining carbon monoxide becomes a catalytic poison in the ammonia synthesis reaction, this carbon monoxide is methanated and removed via a methanation reaction shown in Formula (6) below. Additionally the process gas supplied to the process line L₃ may be preheated to a predetermined temperature suited to the synthesis of the ammonia by a heating device (not illustrated) on the process line L₃.

[Chemical Formula 6]

CO + 3H₂ → CH₄ + H₂O ...... (6)

After synthesizing the process gas supplied from the process line L₃ into ammonia at the first synthesizer 2c, this ammonia is supplied to the synthesis product supply line L₄. At this time, in the ammonia synthesis reaction, an unreacted gas mainly including hydrogen, nitrogen, and methane is generated. Additionally, in order to prevent the accumulation of inert gas, a predetermined amount of the purge gas must be removed from the unreacted gas. Accordingly, a predetermined amount of the unreacted gas is supplied from the synthesis product supply line L₄ to the process line L₃ to recycle the raw material, and the remainder is supplied from the synthesis product supply line L₄ via the purge gas supply line L₁₈ to the hydrogen separation device 3. An amount of the purge gas supplied to the purge gas supply line L₁₈ can be adjusted by a supply amount adjustment valve (not illustrated) provided on the purge gas supply line.

Next, a process of separating hydrogen from the purge gas is described. Hydrogen is separated from the purge gas supplied from the purge gas supply line L₁₈ at the hydrogen separation device 3 juxtaposed with the ammonia manufacturing plant. The separated hydrogen is supplied to the aromatic compound manufacturing device 4 via the hydrogen supply line L₆ for catalyst regeneration, and a portion of the purge gas from which the hydrogen has been separated is supplied to the synthesis gas manufacturing device 2a via the fuel supply line L₇.

Hydrogen is separated from the purge gas at the hydrogen separation device 3. While hydrogen is separated from the purge gas, it is difficult to separate all of the hydrogen. Hydrogen partial pressure is higher in the unreacted gas supplied from the unreacted gas supply line L₅ than the mixed gas from the aromatic compound manufacturing device 4. As such, the hydrogen can be efficiently separated from the unreacted gas, which has high hydrogen partial pressure, generated from the manufacturing device 2 of the ammonia manufacturing plant, and used in the catalyst regeneration at the aromatic compound manufacturing device 4.

Examples of the hydrogen separation method include PSA methods, membrane separation methods, and the like.

Next, a process of manufacturing the aromatic compound will be described. An aromatic compound having six or more carbons, particularly BTX is produced from natural gas mainly including a lower hydrocarbon having four or less carbons, namely methane. Additionally, a mixed gas mainly including unreacted methane and by-product hydrogen is supplied to the manufacturing device 2, and this mixed gas is used as fuel for the ammonia manufacturing process. At this time, the hydrogen supplied from the hydrogen separation device 3 via the hydrogen supply line L₆ to the aromatic compound manufacturing device 4 is used for catalyst regeneration to remove the sediment accumulated on the catalyst.

Next, a detailed description will be given of a manufacturing process of the aromatic compound. As illustrated in FIG. 2, after the heater 4a preheats the natural gas supplied from the raw-material supply line L₈ to a predetermined temperature suited to the synthesis of the aromatic compound at the heater 4a, this preheated natural gas is supplied to the process line L₄₁. Heat exchange to the natural gas from the heat of a process gas supplied via the process line L₄₂ from the second synthesizer 4b is performed and, after using the residual heat of the methane gas, this natural gas is supplied to the process line L₄₃. Thereby, the preheating of the fuel supplied from the raw-material supply line L₈ can be efficiently carried out.

An aromatic compound having six or more carbons, particularly BTX is synthesized from the methane supplied from the process line L₄₁ via a catalytic reaction using a predetermined catalyst in the second synthesizer 4b, and this aromatic compound is supplied to the process line L₄₂. As this time, a portion of the mixed gas including the unreacted methane and the by-product hydrogen supplied via the mixed gas supply line L₁₀ is combusted in a heating furnace (not illustrated). Additionally, hydrogen gas supplied from the hydrogen supply line L₆ is supplied to a catalyst provided in the second synthesizer 4b for catalyst regeneration.

The reaction mechanisms of the reaction by which the BTX is synthesized and particularly a MTB reaction where benzene is synthesized from methane, includes a reaction mechanism in which carbon is generated as a by-product because the hydrocarbon is subjected to a selective dehydrogenation reaction. The carbon generated in this way mainly deposits and accumulates on the catalyst surface and, as a result, deterioration of the catalyst advances with time, and the catalytic activity thereof declines. As a countermeasure, hydrogen gas for catalyst regeneration is supplied to the second synthesizer 4b from the hydrogen separation device 3. The hydrogen gas is circulated and removes the carbon that has accumulated on the catalyst surface. Thus, declines in the catalytic activity can be prevented by using the supplied hydrogen gas as a catalyst regeneration gas. Note that the catalyst regeneration may be performed simultaneously with the synthesis reaction of the BTX, or may be performed intermittently by stopping the raw material supply from the raw-material supply line L₈ and only supplying the hydrogen.

In the synthesis reaction of the BTX and particularly the MTB reaction where benzene is synthesized from methane, as reaction conditions, an internal pressure is preferably not lower than 0.1 MPa and not higher than 3.0 MPa, and an internal temperature is preferably not lower than 600°C and not higher than 1,000°C and more preferably not lower than 700°C and not higher than 900°C. Under these reaction conditions, the manufacturing efficiency of the BTX and the conversion rate of the methane under low reaction pressure can be improved. By obtaining the energy source needed for the BTX manufacturing from the energy source generating facilities of the ammonia manufacturing plant, the facilities will increase in size, and the co-production efficiency of the ammonia and the BTX can be improved.

A catalyst, in which an active metal is supported on a support, is disposed as the catalyst used in the catalytic reaction. Zeolite, silica, alumina, titania, zirconia, ceria, or a combination of these can be used as the support. Molybdenum (Mo), cobalt (Co), zinc (Zn), gallium (Ga), iron (Fe), copper (Cu), silver (Ag), nickel (Ni), tungsten (W), rhenium (Re), barium (Ba), manganese (Mn), zirconium (Zr), platinum (Pt), ruthenium (Ru), rhodium (Rh), palladium (Pd), or a combination of these can be used as the active metal. The support is preferably a zeolite and more preferably a ZSM-5 type zeolite, and the active metal is preferably molybdenum. With this type of catalyst, the manufacturing efficiency of the BTX and the conversion rate of the methane under low reaction pressure can be improved. As a result, the co-production efficiency of the ammonia and the BTX can be improved.

The pressure of the process gas supplied from the process line L₄₃ is increased at the second compressor 4c and this process gas is supplied to the process line L₄₅ in order to separate/recover the liquid phase BTX from the process gas supplied from the process line L₄₃ at the gas-liquid separator 4e, preferably such that a recovery rate of the BTX is about 80% or greater. Note that the process gas supplied from the process line L₄₃ to the second compressor 4c has been cooled to a predetermined temperature by a cooler (not illustrated).

The process gas supplied from the process line L₄₅ is further cooled in the cooler 4d to facilitate the separation/recovery of the liquid phase BTX, and this cooled process gas is supplied to the process line L₄₆. An energy source generated at the ammonia manufacturing plant can also be used at the second compressor 4c and the cooler 4d. From the perspective of co-production efficiency, the ammonia obtained from the cooling system of the ammonia manufacturing plant described above is preferably diverted and used as a cooling source.

Examples of a cooling medium used in the cooling include organic cooling mediums such as methanol (CH₄O), ethylene glycol (C₂H₆O₂), ammonia (NH₃), and the like; flammable chlorofluorocarbon-based solvents such as HFC-32 (CH₂F₂) and the like; and non-flammable chlorofluorocarbon-based solvents such as HFC-23 (CHF₃), HFC-134a (CH₂FCF₃), HCFC-22 (CHClF₂), HCFC-124 (CHClCF₃), PFC-14 (CF₄), PFC-116 (C₂F₆), PFC-218 (C₃F₈), and the like. From the perspective of manufacturing costs, a product of the juxtaposed plant is preferably used as the cooling medium. For example, in the present embodiment, because an ammonia manufacturing plant is juxtaposed, the cooling medium is preferably ammonia.

The process gas supplied from the process line L₄₆ is separated into liquid phase BTX and gas phase mixed gas at the gas-liquid separator 4e; and the liquid phase BTX is supplied to the BTX supply line L₉, and the gas phase mixed gas is supplied to the mixed gas supply line L₁₀. Note that the mixed gas supply line L₁₀ branches so that a portion of the mixed gas mainly including unreacted methane and by-product hydrogen is supplied to the second synthesizer 4b, and the remainder is supplied to the synthesis gas manufacturing device 2a. Additionally, an amount of the mixed gas supplied to the second synthesizer 4b can be adjusted by a supply amount adjustment valve (not illustrated) provided on the mixed gas supply line L₁₀.

According to the present embodiment, at the ammonia manufacturing plant where ammonia is synthesized from natural gas mainly including methane, the large amount of surplus hydrogen included in the unreacted material, namely the purge gas, of the ammonia synthesis reaction in the manufacturing device 2 can be used for the regeneration of the catalyst used in the catalytic reaction in the aromatic compound manufacturing device 4; and the unreacted gas of the aromatic compound manufacturing device 4 can be used as the fuel for the steam-reforming reaction in the manufacturing device 2. Accordingly, it is possible to synthesize and manufacture both the ammonia and the BTX, and efficiently obtain ammonia and BTX with suppressed manufacturing costs.

Additionally, the surplus energy sources of the ammonia manufacturing plant can be used at the second compressor 4c and/or the cooler 4d for the synthesis of the aromatic compound. Thereby, energy source costs and maintenance costs of the facilities can be suppressed and the co-production efficiency of the BTX and the ammonia can be improved.

Furthermore, the large cooling system provided in the manufacturing facilities 4 of the ammonia manufacturing plant can be used at the cooler 4d of the aromatic compound synthesis device 2. As a result, manufacturing costs can be suppressed and the co-production efficiency of the BTX and the ammonia can be improved.

Hereinafter, a second embodiment of the system for manufacturing an aromatic compound according to the present invention will be described in detail while referring to the attached drawings. Note that configurations that are the same in the first embodiment of the system for manufacturing an aromatic compound are omitted from the description of the second embodiment.

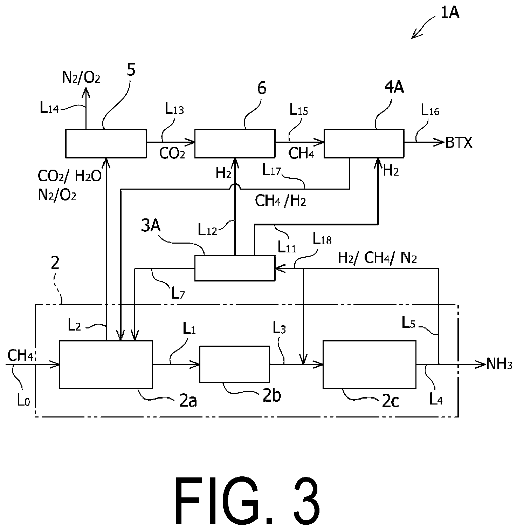

FIG. 3 is a conceptual drawing explaining the second embodiment of the system for manufacturing an aromatic compound according to the present invention. As illustrated in FIG. 3, the system for manufacturing an aromatic compound 1A of the present embodiment includes a manufacturing device 2 (first manufacturing device), a hydrogen separation device 3A, an aromatic compound manufacturing device 4A (second manufacturing device), a carbon dioxide recovery device 5, and a methane manufacturing device 6 (third manufacturing device).

The manufacturing device 2 is provided in an ammonia manufacturing plant that synthesizes ammonia from natural gas that mainly includes methane. Additionally, the hydrogen separation device 3A, the aromatic compound manufacturing device 4A, the carbon dioxide recovery device 5, and the methane manufacturing device 6 are juxtaposed with the ammonia manufacturing plant.

The hydrogen separation device 3A is provided juxtaposed with the ammonia manufacturing plant, and is connected to a purge gas supply line L₁₈, a fuel supply line L₇, and hydrogen supply lines L₁₁ and L₁₂. Purge gas supplied at a predetermined amount via the purge gas supply line L₁₈ is supplied to the hydrogen separation device 3A to prevent the accumulation of inert gas. The hydrogen separation device 3A is configured to separate hydrogen from the purge gas supplied from the purge gas supply line L₁₈, supply a portion of the hydrogen to the aromatic compound manufacturing device 4A via the hydrogen supply line L₁₁, and supply the remainder of the hydrogen to the methane manufacturing device 6 via the hydrogen supply line L₁₂ and also to the manufacturing device 2 via the fuel supply line L₇.

While hydrogen is separated from the purge gas at the hydrogen separation device 3A, it is difficult to separate all of the hydrogen. Hydrogen partial pressure is higher in the unreacted gas supplied from the purge gas supply line L₁₈ through the unreacted gas supply line L₅ than the mixed gas from the aromatic compound manufacturing device 4A. As such, the hydrogen can be efficiently separated from the unreacted gas, which has high hydrogen partial pressure, generated from the manufacturing device 2 of the ammonia manufacturing plant; and can be used in the catalyst regeneration at the aromatic compound manufacturing device 4A and in methane synthesis at the methane manufacturing device 6.

The same method used in the first embodiment can be used here as the hydrogen separation method in the hydrogen separation device 3A.

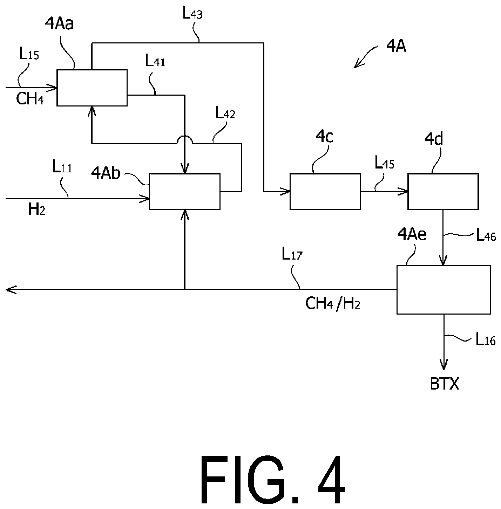

FIG. 4 is a conceptual drawing explaining the aromatic compound manufacturing device 4A of the second embodiment of the system for manufacturing an aromatic compound and the method for manufacturing the aromatic compound according to the present invention. As illustrated in FIG. 4, the aromatic compound manufacturing device (second manufacturing device) 4A includes a heater 4Aa, a second synthesizer 4Ab, a second compressor 4c, a cooler 4d, and a gas-liquid separator 4Ae. The aromatic compound manufacturing device 4A is configured to produce an aromatic compound having six or more carbons, particularly BTX, from natural gas mainly including a lower hydrocarbon having four or less carbons, namely methane. The aromatic compound manufacturing device 4A is configured to supply a mixed gas mainly including unreacted methane and by-product hydrogen to the manufacturing device 2, and use this mixed gas as fuel for the ammonia manufacturing process. Additionally, the aromatic compound manufacturing device 4A is configured to remove sediment from the surface of the catalyst used in the aromatic compound, using the hydrogen supplied from the hydrogen separation device 3A.

The heater 4Aa is connected to the methane gas supply line L₁₅ and process lines L₄₁ to L₄₃. The heater 4Aa is configured to preheat the methane gas supplied from the methane gas supply line L₁₅ to a predetermined temperature suited to the synthesis of the aromatic compound, and supply this preheated methane gas to the process line L₄₁. Additionally, from the perspective of manufacturing efficiency, the heater 4Aa is configured to cause heat exchange to the methane gas from the heat of a process gas with an elevated temperature supplied via the process line L₄₂ from the second synthesizer 4Ab described hereinafter, use the residual heat of the methane gas, and supply this methane gas to the process line L₄₃. Thereby, the preheating of the raw material of the BTX supplied from the methane gas supply line L₁₅ can be efficiently carried out.

The second synthesizer 4Ab is connected to the process lines L₄₁ and L₄₂, the mixed gas supply line L₁₇, and the hydrogen supply line L₁₁. The second synthesizer 4Ab is configured to synthesize an aromatic compound having six or more carbons, particularly BTX, from the methane supplied from the process line L₄₁, and supply the aromatic compound to the process line L₄₂. Additionally, the second synthesizer 4Ab is configured to combust a portion of the mixed gas including the unreacted methane and the by-product hydrogen supplied via the mixed gas supply line L₁₇ in a heating furnace (not illustrated). Furthermore, the second synthesizer 4Ab is configured to supply hydrogen gas, which is supplied from the hydrogen supply line L₁₁, to a catalyst provided therein for catalyst regeneration.

The reaction mechanisms of the reaction by which the BTX is synthesized in the second synthesizer 4Ab and particularly a MTB reaction where benzene is synthesized from methane, includes a reaction mechanism in which carbon is generated as a by-product because the hydrocarbon is subjected to a selective dehydrogenation reaction. The carbon generated in this way mainly deposits and accumulates on the catalyst surface and, as a result, deterioration of the catalyst advances with time, and the catalytic activity thereof declines. As a countermeasure, hydrogen gas for catalyst regeneration is supplied to the second synthesizer 4Ab from the hydrogen separation device 3A. The hydrogen gas is circulated and removes the carbon that has accumulated on the catalyst surface. Thus, declines in the catalytic activity can be prevented by using the supplied hydrogen gas as a catalyst regeneration gas. Note that the catalyst regeneration may be performed simultaneously with the synthesis reaction of the BTX, or may be performed intermittently by stopping the raw material supply from the methane gas supply line L₁₅ and only supplying the hydrogen.

The catalyst disposed in the second synthesizer 4Ab may be the same as the catalyst used in the first embodiment.

The conditions when the catalytic reaction in the second synthesizer 4Ab is carried out may be the same as the conditions imposed in the first embodiment.

The gas-liquid separator 4Ae is connected to the process line L₄₆, a BTX supply line L₁₆, and the mixed gas supply line L₁₇. The gas-liquid separator 4Ae is configured to separate the process gas supplied from the process line L₄₆ into liquid phase BTX and gas phase mixed gas mainly including unreacted methane and by-product hydrogen, supply the liquid phase BTX to the BTX supply line L₁₆, and supply the gas phase mixed gas to the mixed gas supply line L₁₇. Note that the mixed gas supply line L₁₇ is configured to branch so that a portion of the mixed gas is supplied to the second synthesizer 4Ab, and the remainder is supplied to the synthesis gas manufacturing device 2a. Additionally, an amount of the mixed gas supplied to the second synthesizer 4Ab can be adjusted by a supply amount adjustment valve (not illustrated) provided on the mixed gas supply line L₁₇. Thus, the second manufacturing device 4A is configured to manufacture the BTX and supply the mixed gas generated in the synthesis reaction of the BTX to the first manufacturing device 2 as fuel.

A commonly known recovery device juxtaposed with the ammonia manufacturing plant can be used as the carbon dioxide recovery device 5 and, preferably, a recovery device that uses the KM-CDR Process® is used. The carbon dioxide recovery device 5 is connected to the exhaust gas supply line L₂, a carbon dioxide supply line L₁₃, and a treated gas supply line L₁₄. Exhaust gas supplied from the exhaust gas supply line L₂ mainly includes carbon dioxide, water, nitrogen, and oxygen. The carbon dioxide recovery device 5 is configured to separate and recover carbon dioxide from the exhaust gas, and supply this carbon dioxide to the carbon dioxide supply line L₁₃. Additionally, the carbon dioxide recovery device 5 is configured to supply treated gas, obtained by removing the carbon dioxide from the exhaust gas, to the treated gas supply line L₁₄.

The methane manufacturing device 6 is juxtaposed with the ammonia manufacturing plant, has adjustable internal temperature and pressure, and is connected to the hydrogen supply line L₁₂, the carbon dioxide supply line L₁₃, and the methane gas supply line L₁₅. The methane manufacturing device 6 is configured to synthesize methane through a methanation reaction using a catalyst provided therein from the hydrogen supplied from the hydrogen supply line L₁₂ and the carbon dioxide supplied from the carbon dioxide supply line L₁₃, and supply this methane to the methane gas supply line L₁₅.

A commonly known catalyst capable of methanation from carbon dioxide and hydrogen can be used as the catalyst provided in the methane manufacturing device 6. The catalyst is preferably a catalyst made from nickel. The methanation reaction produces methane from carbon dioxide and hydrogen as shown in Formula (7) below. Due to the fact that methanation reactions are exothermic reactions, the catalyst temperature may be controlled using a cooler (not illustrated). In the reaction, typically there is a shortage of hydrogen, but in the ammonia manufacturing process of the present embodiment, the natural gas is supplied from an external source as fuel and also the unreacted gas of the aromatic compound manufacturing device 4A is supplied as fuel. Therefore, a large amount of hydrogen is generated as an unreacted material. As such, the methane synthesis can be carried out without needing to supplement hydrogen from an outside source. Note that as water is generated in the reaction, this water may be removed using a gas-liquid separation device (not illustrated) to increase the methane purity of the methane-containing gas supplied to the aromatic compound manufacturing device 4A.

[Chemical Formula 7]

CO₂ + 4H2 → CH₄ + 2H₂O ...... (7)

In the ammonia manufacturing plant where ammonia is synthesized from natural gas, the natural gas supplied to the synthesis device 2 can be supplied from an outside source. According to the present embodiment, the same beneficial effects are provided as in the first embodiment, methane can be synthesized from the surplus hydrogen of an ammonia manufacturing plant and the carbon dioxide to be discharged into the atmosphere as exhaust gas, and this methane can be used in the manufacture of the BTX at the second synthesizer 4Ab of the aromatic compound manufacturing device 4A. Accordingly, the BTX and the ammonia can be efficiently co-produced. Additionally, the present embodiment is preferable from the perspective of global warming prevention as the carbon dioxide that would have been discharged into the atmosphere can be effectively utilized as a raw material.

Next, by describing the operating configuration of the system for manufacturing an aromatic compound (the second embodiment), a second embodiment of the method for manufacturing the aromatic compound according to the present invention will be described in detail. Note that descriptions of configurations that are the same in the first embodiment of the method for manufacturing the aromatic compound are omitted.

A process of separating hydrogen from the purge gas is described. Hydrogen is separated from the purge gas supplied from the purge gas supply line L₁₈ through the unreacted gas supply line L₅ at the hydrogen separation device 3A juxtaposed with the ammonia manufacturing plant. A portion of the separated hydrogen is supplied to the synthesis gas manufacturing device 2a via the fuel supply line L₇, and the remainder is supplied to the aromatic compound manufacturing device 4A via the hydrogen supply line L₁₁ and also is supplied to the methane manufacturing device 6 via the hydrogen supply line L₁₂.

Hydrogen is separated from the purge gas at the hydrogen separation device 3A. Hydrogen partial pressure is higher in the unreacted gas supplied from the unreacted gas supply line L₅ than the unreacted gas from the aromatic compound manufacturing device 4A. As such, the hydrogen can be efficiently separated from the unreacted gas, which has high hydrogen partial pressure, generated from the manufacturing device 2 of the ammonia manufacturing plant; and can be used in the catalyst regeneration at the aromatic compound manufacturing device 4A and in methane synthesis at the methane manufacturing device 6.

The same method used in the first embodiment can be used here as the hydrogen separation method.

Next, a process of manufacturing the aromatic compound will be described. An aromatic compound having six or more carbons, particularly BTX is produced from natural gas mainly including a lower hydrocarbon having four or less carbons, namely methane. Additionally, a mixed gas mainly including unreacted methane and by-product hydrogen is supplied to the manufacturing device 2, and this mixed gas is used as fuel for the ammonia manufacturing process. At this time, the hydrogen supplied from the hydrogen separation device 3 via the hydrogen supply line L₁₁ to the aromatic compound manufacturing device 4A is used for catalyst regeneration to remove the sediment accumulated on the catalyst.

Next, a detailed description will be given of a manufacturing process of the aromatic compound. As illustrated in FIG. 4, after the heater 4Aa preheats the natural gas supplied from the raw-material supply line L₁₅ to a predetermined temperature suited to the synthesis of the aromatic compound at the heater 4Aa, this preheated natural gas is supplied to the process line L₄₁.

An aromatic compound having six or more carbons, particularly BTX is synthesized from the methane supplied from the process line L₄₁ through a catalytic reaction using a predetermined catalyst in the second synthesizer 4Ab, and this aromatic compound is supplied to the process line L₄₂. As this time, a portion of the mixed gas including the unreacted methane and the by-product hydrogen supplied via the mixed gas supply line L₁₇ is combusted in a heating furnace (not illustrated). Additionally, hydrogen gas supplied from the hydrogen supply line L₁₁ is supplied to a catalyst provided in the second synthesizer 4Ab for catalyst regeneration. Heat exchange to the natural gas from the heat of a process gas supplied via the process line L₄₂ from the second synthesizer 4Ab is performed and, after using the residual heat of the methane gas, this natural gas is supplied to the process line L₄₃. Thereby, the preheating of the raw material supplied from the raw-material supply line L₁₅ can be efficiently carried out.

The reaction mechanisms of the reaction by which the BTX is synthesized and particularly a MTB reaction where benzene is synthesized from methane, includes a reaction mechanism in which carbon is generated as a by-product because the hydrocarbon is subjected to a selective dehydrogenation reaction. The carbon generated in this way mainly deposits and accumulates on the catalyst surface and, as a result, deterioration of the catalyst advances with time, and the catalytic activity thereof declines. As a countermeasure, hydrogen gas for catalyst regeneration is supplied to the second synthesizer 4Ab from the hydrogen separation device 3A. The hydrogen gas is circulated and removes the carbon that has accumulated on the catalyst surface. Thus, declines in the catalytic activity can be prevented by using the supplied hydrogen gas as a catalyst regeneration gas. Note that the catalyst regeneration may be performed simultaneously with the synthesis reaction of the BTX, or may be performed intermittently by stopping the raw material supply from the raw-material supply line L₁₅ and only supplying the hydrogen.

The same catalyst used in the first embodiment can be used as the catalyst in this type of BTX synthesis reaction, particularly in the MTB reaction where benzene is synthesize from methane.

The same reaction conditions used in the first embodiment can be imposed here as the conditions for the reaction using this catalyst.

The process gas supplied via the process lines L₄₃ to L₄₆, the second compressor 4c, and the cooler 4d is separated at the gas-liquid separator 4Ae into liquid phase BTX and gas phase mixed gas. The separated BTX is supplied to the BTX supply line L₁₆, and the mixed gas is supplied to the mixed gas supply line L₁₇. Additionally, the mixed gas supply line L₁₇ branches so that a portion of the mixed gas mainly including unreacted methane and by-product hydrogen is supplied to the second synthesizer 4Ab, and the remainder is supplied to the synthesis gas manufacturing device 2a. Additionally, an amount of the mixed gas supplied to the second synthesizer 4Ab can be adjusted by a supply amount adjustment valve (not illustrated) provided on the mixed gas supply line L₁₇.

Next, a process of synthesizing the methane is described. Exhaust gas supplied from the exhaust gas supply line L₂ mainly includes water, nitrogen, and oxygen, in addition to carbon dioxide. As such, the carbon dioxide is separated and recovered from the exhaust gas at the carbon dioxide recovery device 5, and supplied to the carbon dioxide supply line L₁₃. Additionally, treated gas, obtained by removing the carbon dioxide from the exhaust gas, is supplied to the treated gas supply line L₁₄. A KM-CDR Process® is preferably used as the carbon dioxide recovery device 5.

Methane is synthesized in the methane manufacturing device 6 through a methanation reaction using a catalyst, from the hydrogen supplied from the hydrogen supply line L₁₂ and the carbon dioxide supplied from the carbon dioxide supply line L₁₃, and is supplied to the methane gas supply line L₁₅.

A commonly known catalyst capable of methanation from carbon dioxide and hydrogen can be used as the catalyst used in the methanation reaction. The catalyst is preferably a catalyst made from nickel. The methanation reaction produces methane from carbon dioxide and hydrogen as shown in Formula (7) below. Due to the fact that methanation reactions are exothermic reactions, the catalyst temperature may be controlled using a cooler (not illustrated). In the reaction, typically there is a shortage of hydrogen, but in the ammonia manufacturing process of the present embodiment, the natural gas is supplied from an external source as fuel and also the unreacted gas of the aromatic compound manufacturing device 4A is supplied as fuel. Therefore, a large amount of hydrogen is generated as an unreacted material. As such, the methane synthesis can be carried out without needing to supplement hydrogen from an outside source. Note that as water is generated in the reaction, this water may be removed using a gas-liquid separation device (not illustrated) to increase the methane purity in the gas supplied to the aromatic compound manufacturing device 4A.

[Chemical Formula 8]

CO₂ + 4H2 → CH₄ + 2H₂O ...... (7)

In the ammonia manufacturing plant where ammonia is synthesized from natural gas, the natural gas supplied to the synthesis device 2 can be supplied from an outside source. According to the present embodiment, the same beneficial effects are provided as in the first embodiment, methane can be synthesized from the surplus hydrogen of an ammonia manufacturing plant and the carbon dioxide to be discharged into the atmosphere as exhaust gas, and this methane can be used in the manufacture of the BTX at the second synthesizer 4Ab of the aromatic compound manufacturing device 4A. Accordingly, the BTX and the ammonia can be efficiently co-produced. Additionally, the present embodiment is preferable from the perspective of global warming prevention as the carbon dioxide that would have been discharged into the atmosphere can be effectively utilized as a raw material.

Note that in the embodiments described above, examples were given in which the hydrogen separation devices 3 and 3A and the aromatic compound manufacturing devices 4 and 4A were juxtaposed with the ammonia manufacturing plant, but the present invention is not limited to this configuration. For example, from the perspectives of manufacturing investment costs, motive power, and maintenance costs, the hydrogen separation devices 3 and 3A and/or the aromatic compound manufacturing devices 4 and 4A may be disposed within the ammonia manufacturing plant. In such a case, the energy sources of the ammonia manufacturing plant can be used. Additionally, the hydrogen separation devices 3 and 3A and/or the aromatic compound manufacturing devices 4 and 4A may be provided in a separate or newly constructed aromatic compound manufacturing plant. In such a case, a portion of the energy sources generated at the ammonia manufacturing plant can be used at the hydrogen separation devices 3 and 3A and/or the aromatic compound manufacturing devices 4 and 4A, and a portion of the energy source generated at the aromatic compound manufacturing plant can be used at the manufacturing device 2. These configurations contribute greatly to the improvement of the co-production efficiency of the ammonia and the aromatic compound.

In the embodiments described above, examples were given in which the carbon dioxide recovery device 5 and the methane manufacturing device 6 were juxtaposed with the ammonia manufacturing plant, but the present invention is not limited to this configuration. For example, from the perspectives of manufacturing investment costs, motive power, and maintenance costs, the carbon dioxide recovery device 5 and/or the methane manufacturing device 6 may be disposed within the ammonia manufacturing plant. In such a case, the energy sources of the ammonia manufacturing plant can be used. Additionally, the carbon dioxide recovery device 5 and/or the methane manufacturing device 6 may be provided in a separate or newly constructed aromatic compound manufacturing plant. In such a case, a portion of the energy sources generated at the ammonia manufacturing plant can be used at the carbon dioxide recovery device 5 and/or the methane manufacturing device 6, and a portion of the energy source generated at the aromatic compound manufacturing plant can be used at the carbon dioxide recovery device 5 and/or the methane manufacturing device 6. These configurations contribute greatly to the improvement of the co-production efficiency of the ammonia and the aromatic compound.

In the embodiments described above, examples were given in which the manufacturing device 2 for synthesizing the target substance from the natural gas is provided in the ammonia manufacturing plant, but the present invention in not limited to this configuration. Provided that the plant has the same manufacturing facilities or uses the same manufacturing methods as a plant for manufacturing a target substance from natural gas, the manufacturing device 2 can be applied to, for example, a methanol manufacturing plant, a hydrogen manufacturing plant, a urea manufacturing plant, or similar manufacturing device.

In the embodiments described above, examples were given in which natural gas was used as the raw material, but the present invention in not limited to this configuration. Provided that the raw material contains lower hydrocarbons, coal gasification gas, biomass gasification gas, coke oven gas (COG) and the like, for example, may be applied as the raw material. It is possible to co-produce the compound and the aromatic compound from these types of raw materials.

### Industrial Applicability

According to the system for manufacturing an aromatic compound and the method for synthesizing the aromatic compound according to the present invention, the purity of surplus hydrogen in purge gas generated as a by-product of a plant can be increased and used in the regeneration of a catalyst used in the synthesis of an aromatic compound. Additionally, unreacted gas from the synthesis of the aromatic compound can be used at an ammonia or methanol manufacturing plant. Furthermore, as a result of juxtaposing the ammonia or methanol manufacturing plant with the aromatic compound manufacturing device, the aromatic compound and the product of the ammonia or methanol manufacturing plant can be efficiently co-produced.

### Reference Signs List

- 1, 1A: System for manufacturing an aromatic compound
- 2: Manufacturing device (first manufacturing device)
- 2a: Steam reformer
- 2b: First compressor
- 2c: First synthesizer
- 3, 3A: Hydrogen separation device
- 4, 4A: Aromatic compound manufacturing device (second manufacturing device)
- 4a, 4Aa: Heater
- 4b, 4Ab: Second synthesizer
- 4c: Second compressor
- 4d: Cooler
- 4e, 4Ae: Gas-liquid separator
- 5: Carbon dioxide recovery device
- 6: Methane manufacturing device (third manufacturing device)

## Claims

1. A system for manufacturing an aromatic compound, the system comprising:
a first manufacturing device that synthesizes a target substance from natural gas;
a second manufacturing device that synthesizes an aromatic compound from the natural gas via a catalytic reaction, and supplies a mixed gas mainly including unreacted methane and by-product hydrogen to the first manufacturing device to manufacture the target substance; and
a hydrogen separation device that separates hydrogen from purge gas generated from a synthesis reaction of the first manufacturing device, and supplies the hydrogen to the second manufacturing device to regenerate a catalyst used in the catalytic reaction.

2. The system for manufacturing an aromatic compound according to claim 1, further comprising:
a third manufacturing device that synthesizes methane via a methanation reaction from carbon dioxide recovered from exhaust gas of the first manufacturing device and the hydrogen supplied from the hydrogen separation device, and supplies the methane to the second manufacturing device as a raw material.

3. The system for manufacturing an aromatic compound according to claim 1 or 2, wherein:
the target substance is ammonia;
the aromatic compound is one or more types of aromatic compound selected from the group consisting of benzene, toluene, xylene and naphthalene;
the first manufacturing device is installed in an ammonia manufacturing plant; and
the second manufacturing device is juxtaposed with the ammonia manufacturing plant, and uses an energy source of the plant to manufacture the aromatic compound.

4. The system for manufacturing an aromatic compound according to claim 3, wherein the second manufacturing device further comprises a compressor and a cooler for manufacturing the aromatic compound.

5. The system for manufacturing an aromatic compound according to any one of claims 1 to 4, wherein:
the catalyst is a catalyst formed from a ZSM-5 type zeolite;
an internal pressure of the second manufacturing device is not lower than 0.1 MPa and not higher than 3.0 MPa; and
an internal temperature of the second manufacturing device is not lower than 700°C and not higher than 900°C.

6. A method for manufacturing an aromatic compound, the method comprising the steps of:
synthesizing a target substance from natural gas using a first manufacturing device;
synthesizing an aromatic compound from the natural gas via a catalytic reaction using a second manufacturing device, and supplying a mixed gas mainly including unreacted methane and by-product hydrogen to the first manufacturing device to manufacture the target substance; and
separating hydrogen from purge gas generated from the first manufacturing device, and supplying the hydrogen to the second manufacturing device to regenerate a catalyst used in the catalytic reaction.

7. The method for manufacturing an aromatic compound according to claim 6, further comprising a step of:
synthesizing methane via a methanation reaction using a third manufacturing device from carbon dioxide recovered from exhaust gas of the synthesizing step of the first manufacturing device and the hydrogen separated from the purge gas generated from the synthesizing step of the target substance, and supplying the methane to the synthesizing step of the aromatic compound as a raw material.

8. The method for manufacturing an aromatic compound according to claim 6 or 7, wherein:
the target substance is ammonia;
the aromatic compound is one or more types of aromatic compound selected from the group consisting of benzene, toluene, xylene and naphthalene;
the first manufacturing device is an ammonia manufacturing plant; and
the second manufacturing device is juxtaposed with the ammonia manufacturing plant, and uses an energy source of the plant to manufacture the aromatic compound.

9. The method for manufacturing an aromatic compound according to claim 8, wherein:
the step of synthesizing the aromatic compound further comprises a compressing step and a cooling step; and
the compressing step and the cooling step are performed using the energy source of the ammonia manufacturing plant.

10. The method for manufacturing an aromatic compound according to any one of claims 6 to 9, wherein:
the synthesis reaction of the aromatic compound is performed using a catalyst formed from a ZSM-5 type zeolite; and
the synthesis reaction of the aromatic compound is carried out at a pressure of not lower than 0.1 MPa and not higher than 3.0 MPa, and a temperature of not lower than 700°C and not higher than 900°C.
